# EUROPEAN PATENT APPLICATION

(11) **EP 4 772 245 A2**
(43) Date of publication of application: **08.07.2026**
(21) Application number: 26162721.0
(22) Date of filing: 02.01.2020
(51) Int. Cl.: A61P 27/02

(54) **METHOD FOR TREATING LOWER BACK PAIN**

(30) Priority: 02.01.2019 US 201962787654 P
(62) Divisional of application: 20701503.3
(71) Applicant: Mesoblast International Sàrl, 1217 Meyrin (CH)
(72) Inventor: ITESCU, Silviu, Melbourne, 3000 (AU); BROWN, Roger, New York, 10017 (US)
(74) Representative: Thomann, William John

(57) **Abstract**

A method of treating lower back pain in a subject in need thereof, the method comprising administering to the subject a composition comprising mesenchymal lineage precursor or stem cells (MLPSCs), wherein the lower back pain is associated with an intervertebral disc that has a disc height that is not substantially reduced compared to that of an adjacent healthy disc in the subject.

## Description

### Technical Field

The present disclosure relates to compositions comprising mesenchymal lineage precursor or stem cells for the treatment of lower back pain.

### Background

Lower back pain is a chronic condition associated with inflammation This condition affects approximately two-thirds of the U.S. adult population, leads to significant increases in physician office visits, and has a significant effect on disability.

Lumbar disc degeneration, which manifests principally as low back pain, is a substantial social and economic burden to the community. The condition is associated with increases in long-term physical disability and reduction in quality of life. An estimated 80% of the population experiences at least 1 significant episode of low back pain during a lifetime, and approximately 2.5% of the working population will take some sick leave during the year because of low back pain. The direct costs of low back pain in modern western countries is estimated in the billions of dollars, most of which are spent on consulting general practitioners, physical therapists, and other conservative practitioners.^{2,3} Total indirect expenditure, including surgical management, may be 10 times higher.⁴

Symptoms of low back pain often resolve spontaneously as subjects modify their lifestyles to accommodate restricted mobility. Many cases, however, require surgical intervention, with the "gold standard" being spinal fusion to immobilize the 1 or more painful levels. Long-term studies suggest that fusion may actually promote degeneration at adjacent levels.⁵ Non-unions may also occur, and subjects undergoing a repeat surgical fusion may still experience fusion failure.

Oxycodone, morphine and oxymorphone have been used in clinical studies of patients with chronic back pain. Oxycodone controlled release and oxycodone immediate release compositions have been used in clinical studies of patients with stable, chronic moderate-to-severe low back pain as described by Hale et al., Clin. J. Pain, 15, 179-183 (1999). A morphine sulfate extended-release product identified as AVINZA^{®} (Ligand Pharmaceuticals Incorporated, San Diego, Calif., USA) has been approved for once daily administration and is indicated for relief of moderate to severe pain requiring continuous around-the-clock opioid therapy for an extended period of time. An oxymorphone extended release composition has been used in clinical studies of ambulatory patients with moderate-to-severe chronic low back pain as described by Hale et al., Clin. J. Pain, 6(1), 21-28 (2005).

Although a variety of therapeutic agents have been used for treating pain and/or inflammation, including chronic pain and/or inflammation, the treatment is often still ineffective. In particular, back pain is often poorly managed or controlled even by the chronic administration of such agents. This may be due to the loss of potency of the agent and/or the development of side effects associated with chronic treatment with the agent.

### Summary of the disclosure

The present disclosure relates to an improved off-the-shelf ex *vivo* expanded allogeneic mesenchymal lineage precursor or stem cell (MLPSC) product which is has been shown to alleviate lower back pain associated with conditions other than substantially degenerated discs. One potential advantage of the product is that administration may provide long term treatment of pain after only a single dose.

Accordingly, the present disclosure provides a method of treating lower back pain in a subject in need thereof, the method comprising administering to the subject a composition comprising mesenchymal lineage precursor or stem cells (MLPSCs), wherein the lower back pain is associated with a condition other than a substantially degenerated disc.

In one embodiment, the lower back pain is associated with an intervertebral disc that has a disc height that is not substantially reduced compared to that of an adjacent healthy disc in the subject.

In one embodiment, the lower back pain is associated with an intervertebral disc that has a disc height loss of <30% compared to that of an adjacent healthy disc in the subject.

It will be appreciated that disc height can be measured by any suitable method. For example, disc height may be assessed by radiographs such as anterioposterior (AP) or lateral radiographs, flexicon and extension radiographs, or by MRI scans.

In one embodiment, the lower back pain is non-radicular in origin, or is not due to stimulation or nerve roots or dorsal root ganglion of a spinal nerve by compression forces.

In one embodiment, the lower back pain is associated with an intervertebral disc herniation up to a 3mm protrusion. In one example there is no radiographic evidence of neurological compression.

In one embodiment, the subject has a visual analog scale (VAS) back pain score >40 prior to treatment. In one In one embodiment, the subject has an Oswestry disability Index (ODI) score >30 prior to treatment.

In one embodiment, the lower back pain is associated with nerve ingrowth into an intervertebral disc.

In one embodiment, the lower back pain is associated with inflammation in an intervertebral disc. In one example, the inflammation is associated with pro-inflammatory monocytes, T cells or other immune cells that secrete pro-inflammatory cytokines.

The nerve ingrowth or inflammation is in the intervertebral disc space, or the nucleus pulposus, or the annulus fibrosis of the intervertebral disc.

In one embodiment the subject has chronic lower back pain. For example, the subject may have had chronic lower back pain for at least 6 months.

In one embodiment, the subject is assessed or selected for treatment on the basis that the subject exhibits one or more of the conditions described above.

In one embodiment, the MLPSCs release TGFβ1 when cultured in an amount of at least about 2800 pg/10⁶ cells, or at least about at 2810 pg/10⁶ cells, or at least about 2820 pg/10⁶ cells, or at least about 2830 pg/10⁶ cells, or at least about 2840 pg/10⁶ cells, or at least about 2850 pg/10⁶ cells, or at least about 2860 pg/10⁶ cells, or at least about 2870 pg/10⁶ cells, or at least about 2880 pg/10⁶ cells, or at least about 2890 pg/10⁶ cells, or at least about 2900 pg/10⁶ cells, or at least about 2910 pg/10⁶ cells, or at least about 2920 pg/10⁶ cells, or at least about 2930 pg/10⁶ cell, or at least about 2940 pg/10⁶ cells, or at least about 2950 pg/10⁶ cells, or at least about 2960 pg/10⁶ cells, or at least about 2970 pg/10⁶ cells, or at least about 2980 pg/10⁶ cells, or at least about 2990 pg/10⁶ cells, or at least about 3000 pg/10⁶ cells.

In one embodiment, the MLPSCs release TGFβ1 when cultured in an amount of at least about an amount of at least about 400 pg/ml, or at least about 405 pg/ml, or at least about 410 pg/ml, or at least about 415 pg/ml, or at least about 420 pg/ml, or at least about 425 pg/ml, or at least about 430 pg/ml, or at least about 435 pg/ml, or at least about 440 pg/ml, or at least about 445 pg/ml, or at least about 450 pg/ml, or at least about 455 pg/ml, or at least about 460 pg/ml, or at least about 465 pg/ml, or at least about 470 pg/ml, or at least about 475 pg/, or at least about 480 pg/ml, or at least about 485 pg/ml, or at least about 490 pg/ml, or at least about 495 pg/ml, or at least about 500 pg/ml.

In one embodiment the composition comprises MLPSCs that have been assayed to determine release of TGFβ1 under culture conditions. In another embodiment, the composition comprises MLPSCs from a population that has been sampled to determine release of TGFβ1 under culture conditions (i.e., the cells in the composition itself have not been assayed to determine release of TGFβ1 under culture conditions).

In one embodiment, the MLPSCs release sufficient TGFβ1 to stimulate collagen production in human annulus fibrous cells *in vitro.* In one embodiment, the MLPSCs release TGFβ1 in an amount sufficient to enhance Sema3A expression in annulus fibrosis cells *in vitro.*

In one embodiment, the isolated population of cells comprises culture-expanded mesenchymal lineage precursor or stem cells. In an alternate embodiment, the isolated population of cells comprises freshly isolated mesenchymal lineage precursor or stem cells.

In one embodiment, the MLPSCs are isolated by immunoselection. In one embodiment, the cells have been immunoselcted for expression of TNAP. In one embodiment, the immunoselected cells co-express TNAP and STRO-1. In one embodiment, the immunoselected cells co-express TNAP and STRO-1^{bright}. In one embodiment the immunoselected cells are culture expanded prior to administration.

In one embodiment the MLPSCs are mesenchymal stem cells. In on embodiment the mesenchymal stem cells are culture expanded prior to administration.

In one embodiment, the MLPSCs comprise at least 5%, or at least 10%, or at least 20%, or at least 50%, or at least 60%, or at least 70%, or at least 80%, or at least 90%, or a least 95%, or 100% of the total cell population of the composition.

In one embodiment, the composition comprises MLPSCs and a cryopreservative.

In one embodiment the cryopreservative in the composition is DMSO or Profreeze^{®}.

In one embodiment the composition comprises MLPSCs in 42.5% (v/v) Profreeze^{®}/50% αMEM (v/v)/7.5% (v/v) DMSO.

In one embodiment, the composition further comprises hyaluronan, for example, at least about 0.5% HA or HA salt, at least about 0.6% HA or HA salt, at least about 0.7% HA or HA salt, at least about 0.8% HA or HA salt, at least about 0.9% HA or HA salt, at least about 1% HA or HA salt, at least about 1.5% HA or HA salt, at least about 2% HA or HA salt, at least about 2.5% HA or HA salt, at least about 3% HA or HA salt, at least about 3.5% HA or HA salt, at least about 4% HA or HA salt, at least about 4.5% HA or HA salt, at least about 5% HA or HA salt, at least about 6% HA or HA salt, at least about 7% HA or HA salt, at least about 8% HA or HA salt, at least about 9% HA or HA salt, or at least about 10% HA or HA salt.

In one embodiment, the composition is cryopreserved in 42.5% Profreeze^{™}/50% αMEM/7.5% DMSO.In one embodiment, the composition is cryopreserved in Plasmalyte-A, 25%HSA and DMSO.

In one embodiment, wherein the composition is administered to the subject at a dose of between about 1 x 10⁶ cells to about 20 x 10⁶ cells. In one embodiment, the composition is administered to the subject at a dose of about 6 x 10⁶ cells. In one embodiment, the composition is administered to the subject at a dose of about 18 x 10⁶ cells.

In one embodiment, the composition is administered as a single dose.

In one embodiment, the composition is administered into the nucleus pulposus or the annulus fibrosis of an intervertebral disc.

In one embodiment, administration of the MLSPCs results in at least a 50% reduction in pain as determined by the visual analog scale (VAS) for at least 1 month, or at least 6 months, or at least 12 months, or at least 18 months, or at least 24 months after administration.

In one embodiment the 50% reduction in pain as determined by the (VAS) is achieved without further intervention after administration of the MLPSCs.

In one embodiment, administration of the MLSPCs results in a reduction of at least 15 points as determined by the Oswestry Disability Index (ODI) for at least 1 month, or at least 6 months, or at least 12 months, or at least 18 months, or at least 24 months after administration.

In one embodiment the at least 15 point reduction as determined by the ODI is achieved without further intervention after administration of the MLPSCs.

### Brief Description of Drawings

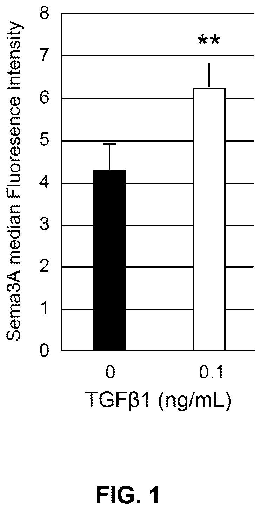

**Figure 1****:** Exposure of human annulus fibrosus cells to recombinant TGFβ1 enhances Sema3A expression as measured by intracellular flow cytometry.

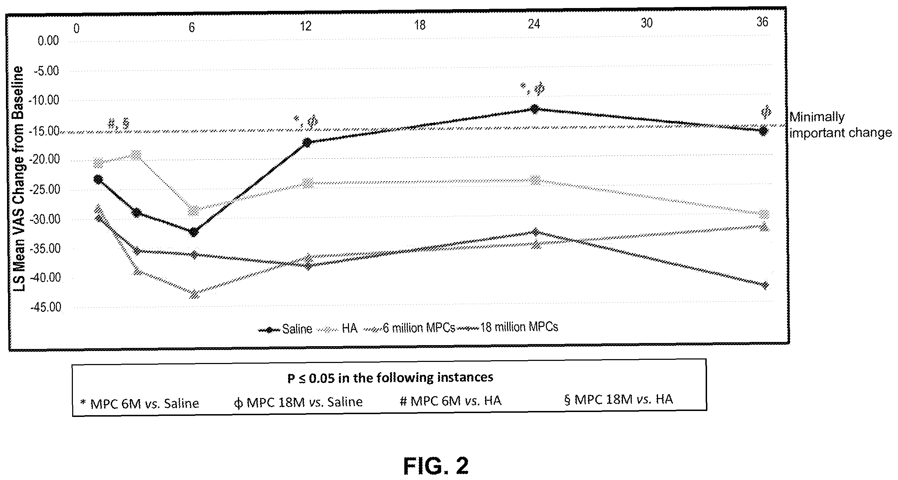

**Figure 2****:** Least squares (LS) Mean VAS lower back pain (LBP) Change from Baseline Scores. Pre-specified LS mean VAS LBP change from baseline analysis was adjusted to remove confounding effects of post-treatment interventions/rescue. Subjects failing therapy due to a post-treatment intervention had baseline observation carried forward (BOCF) imputed for all visits after the intervention. Subjects with missing data at a timepoint with missing data were excluded from the analysis. Ostelo et al (Spine Vol 33,no1.pp90-94) established minimally important changes (MICs) for the most frequently used questionnaires to evaluate pain and function in patients with chronic low back pain.

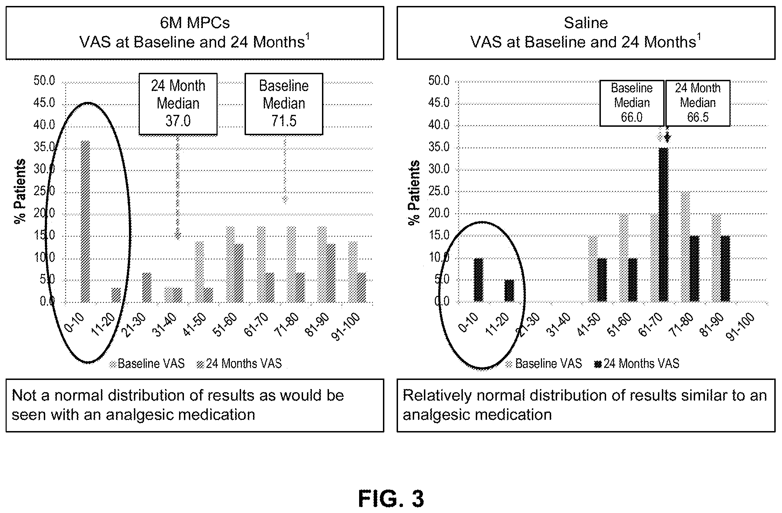

**Figure 3****:** 24 month VAS Categorical Distribution. Subjects failing therapy due to intervention had BOCF imputed for all visits after the intervention. BOCF imputed for patients with missing data.

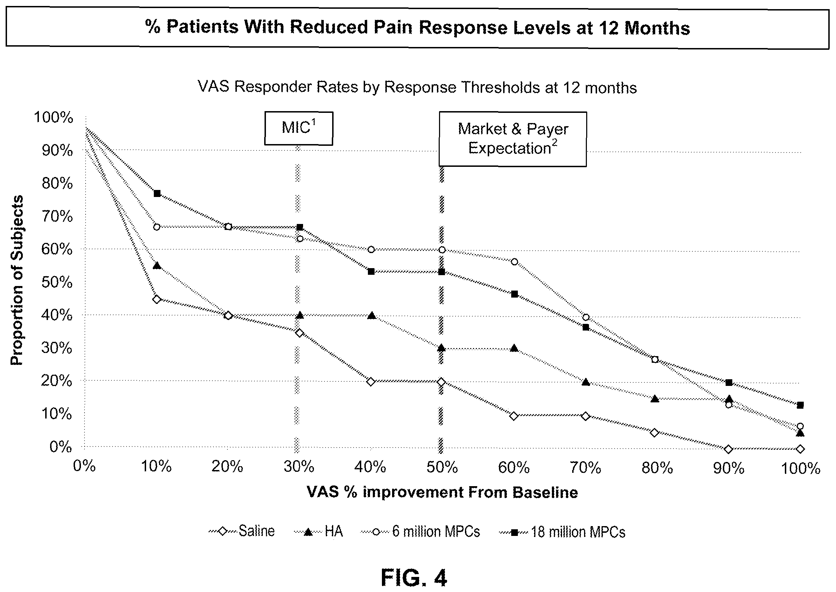

**Figure 4****:** 12 month results. Ostelo et al (Spine Vol 33,no1.pp90-94) established MICs for the most frequently used questionnaires to evaluate pain and function in patients with chronic low back pain. Market & Payer Expectation is based on research conducted by LEK and Navigant for Mesoblast.

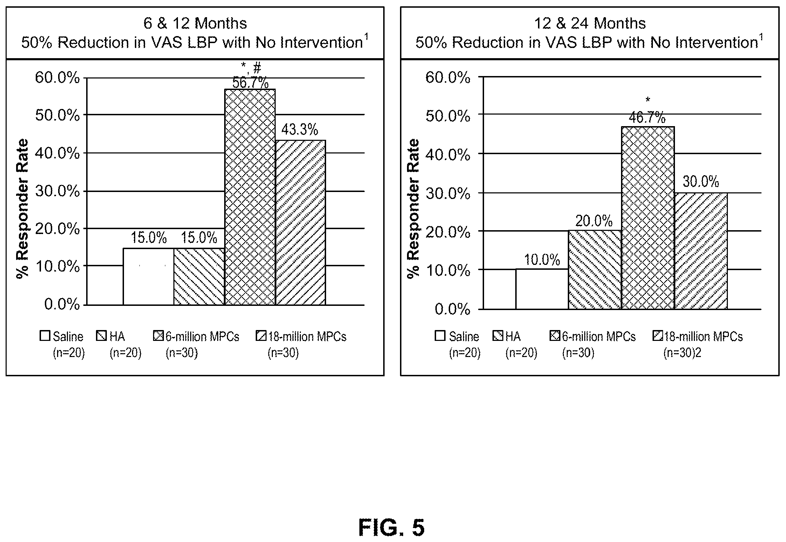

**Figure 5****:** Patients showing 50% reduction in VAS lower back pain with no intervention through 12 and through 24 months. Subjects had to have a 50% reduction in pain measured by VAS at each specified timepoint and no intervention through the latest specified timepoint to be considered a responder. Subjects that were missing were considered a non-responder.

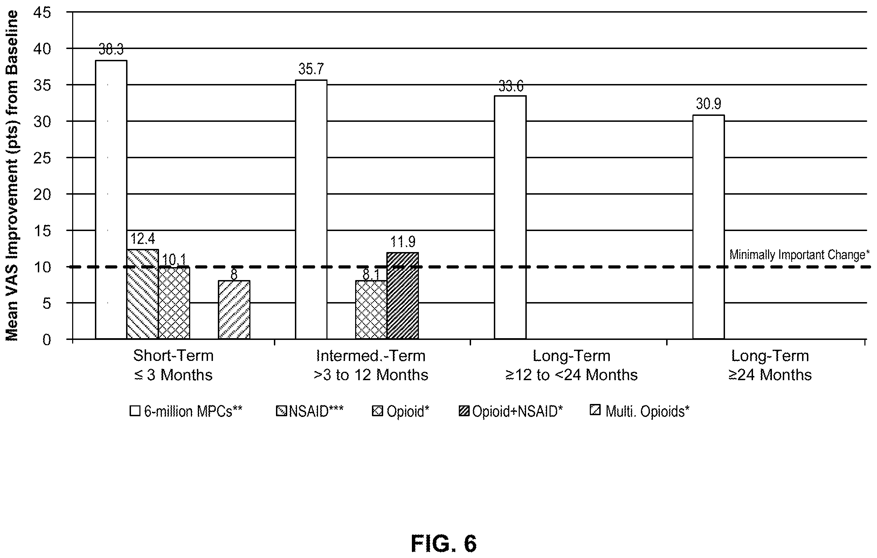

**Figure 6****:** Mean VAS improvement compared to current therapies. Abdel Shaheed Christina, Maher Chris G, Williams Kylie A, Day Richard, McLachlan Andrew J. Efficacy, Tolerability, and Dose--Dependent Effects of Opioid Analgesics for Low Back Pain: A Systema'c Review and Meta---analysis. JAMA Internal Medicine . American Medical Association; 2016 Jul 1;176(7):958-68.
   ** Subjects failing therapy due to a post---treatment intervention had their BOCF imputed for all visits after the intervention. Subjects with missing data were not included in the analyses.
   *** Chou Roger, Deyo Richard, Friedly Janna, Skelly Andrea, Weimer Melissa, Fu Rochelle, Dana Tracy, Kraegel Paul, Griffin Jessica, Grusing Sara. Systemic Pharmacologic Therapies for Low Back Pain: A Systematic Review for an American College of Physicians Clinical Practice Guideline, Annals of internal medicine. American College of Physicians; 2017 Apr 4;166(7):480-492.

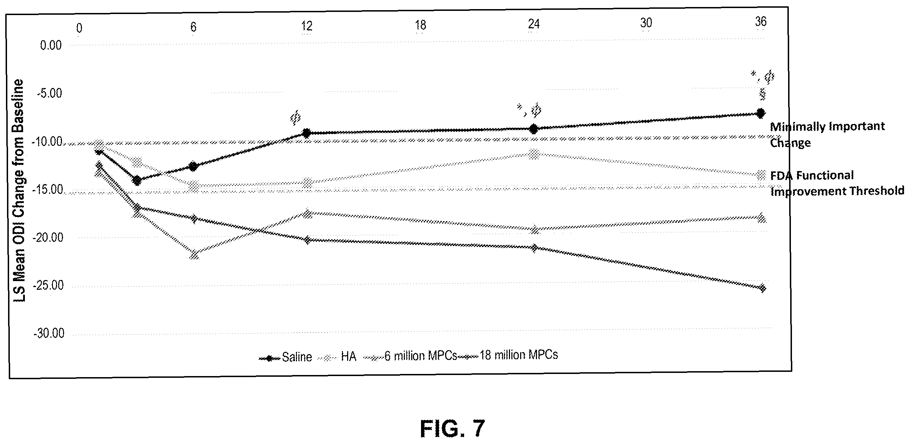

**Figure 7****:** LS mean ODI change from baseline scores. Pre-specified LS mean ODI change from baseline analysis was adjusted to remove confounding effects of post-treatment interventions/rescue. Subjects failing therapy due to a post-treatment intervention had BOCF imputed for all visits after the intervention. Subjects with missing data at a timepoint with missing data were excluded from the analysis. Ostelo et al (Spine Vol 33,no1.pp90-94) established MICs for the most frequently used questionnaires to evaluate pain and function in patients with chronic low back pain.

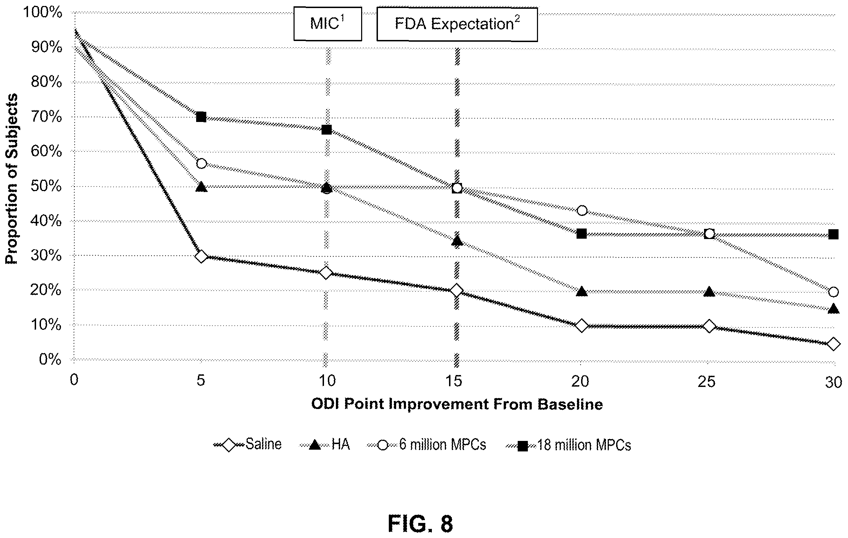

**Figure 8****:** Percentage responders by points of ODI improvement. Ostelo et al. (Spine Vol 33,no1.pp90-94) established MICs for the most frequently used questionnaires to evaluate pain and function in patients with chronic low back pain. FDA has historically required a 15 point improvement in ODI to demonstrate improvement sufficient to support a marketing application for spinal implant devices.

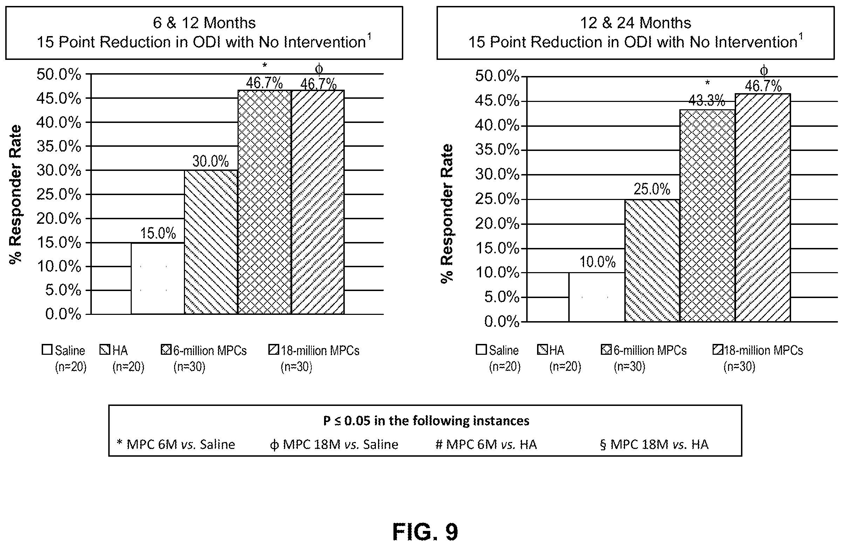

**Figure 9**: Percentage responders showing 15 point reduction in ODI with no intervention through 12 and through 24 months. Subjects had to have a 15 point reduction in ODI score at each specified timepoint and no intervention through the latest specified timepoint to be considered a responder. Subjects that were missing were considered a non-responder.

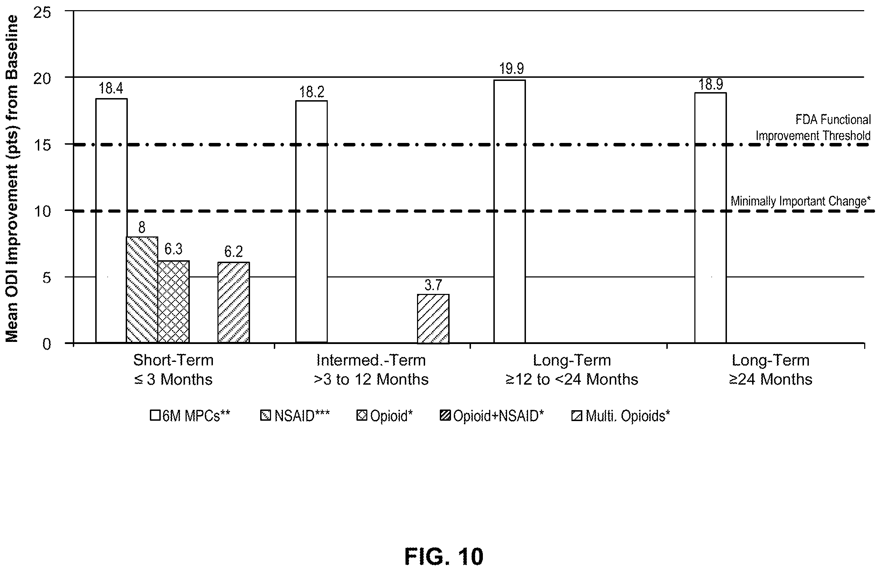

**Figure 10****:** Mean ODI comparison to standard of care.
   *Abdel Shaheed Christina, Maher Chris G, Williams Kylie A, Day Richard, McLachlan Andrew J. Efficacy, Tolerability, and Dose-Dependent Effects of Opioid Analgesics for Low Back Pain: A Systematic Review and Meta-analysis. JAMA Internal Medicine . American Medical Association; 2016 Jul 1;176(7):958-68.
   ** Subjects failing therapy due to a post-treatment intervention had their BOCF imputed for all visits after the intervention. Subjects with missing data were not included in the analyses.
   *** Chou Roger, Deyo Richard, Friedly Janna, Skelly Andrea, Weimer Melissa, Fu Rochelle, Dana Tracy, Kraegel Paul, Griffin Jessica, Grusing Sara. Systemic Pharmacologic Therapies for Low Back Pain: A Systematic Review for an American College of Physicians Clinical Practice Guideline. Annals of internal medicine. American College of Physicians; 2017 Apr 4;166(7):480-492.

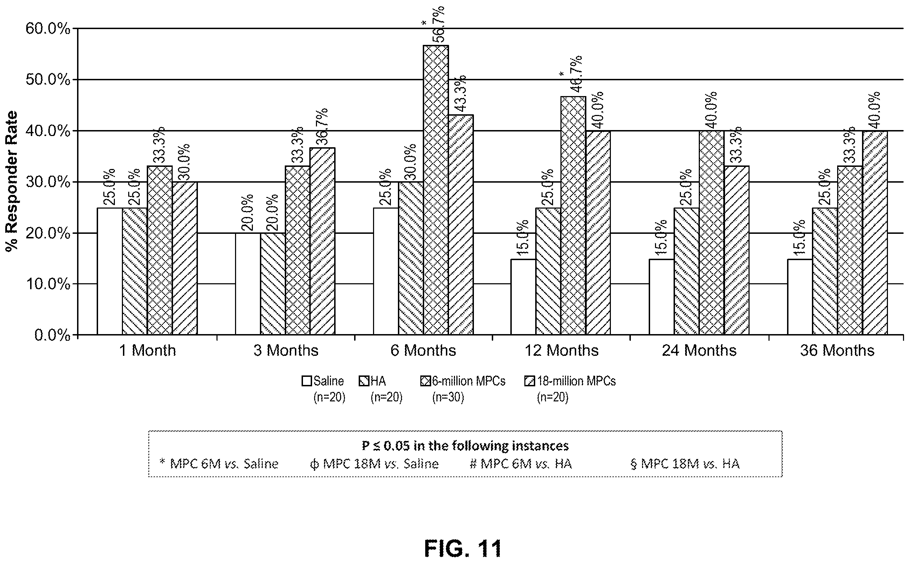

**Figure 11****:** Composite treatment success by timepoint. Subjects with missing data are classified as non-responders. Composite Treatment Success Responders have a 50% reduction in LBP as measured by VAS at the specified timepoint AND a 15 point improvement in function as measured by ODI at the specified timepoint and no intervention through the timepoint specified.

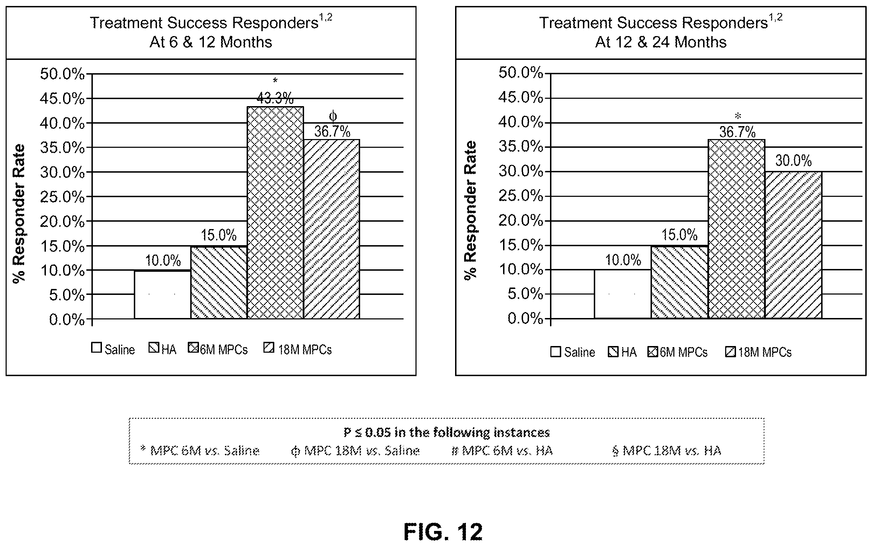

**Figure 12****:** Composite treatment success through 24 months. Subjects with missing data are classified as non-responders. Composite Treatment Success Responders have a 50% reduction in LBP as measured by VAS at the specified timepoint(s) AND a 15 point improvement in function as measured by ODI at the specified timepoint(s) and no intervention through the latest timepoint specified.

### Description of Embodiments

### General techniques and definitions

Throughout this specification, unless specifically stated otherwise or the context requires otherwise, reference to a single step, composition of matter, group of steps or group of compositions of matter shall be taken to encompass one and a plurality (i.e., one or more) of those steps, compositions of matter, group of steps or group of compositions of matter.

Those skilled in the art will appreciate that the disclosure described herein is susceptible to variations and modifications other than those specifically described. It is to be understood that the disclosure includes all such variations and modifications. The disclosure also includes all of the steps, features, compositions and compounds referred to or indicated in this specification, individually or collectively, and any and all combinations or any two or more of said steps or features.

The present disclosure is not to be limited in scope by the specific embodiments described herein, which are intended for the purpose of exemplification only. Functionally-equivalent products, compositions and methods are clearly within the scope of the disclosure.

Any example disclosed herein shall be taken to apply *mutatis mutandis* to any other example unless specifically stated otherwise.

Unless specifically defined otherwise, all technical and scientific terms used herein shall be taken to have the same meaning as commonly understood by one of ordinary skill in the art (e.g., in cell culture, molecular genetics, stem cell differentiation, immunology, immunohistochemistry, protein chemistry, and biochemistry).

Unless otherwise indicated, the stem cells, cell culture, and surgical techniques utilized in the present disclosure are standard procedures, well known to those skilled in the art. Such techniques are described and explained throughout the literature in sources such as Perbal, 1984; Sambrook & Green, 2012; Brown, 1991; Glover & Hames, 1995 and 1996; Ausubel., 1987 including all updates untill present; Harlow & Lane, 1988; and Coligan et al., 1991 including all updates until present.

As used in this specification and the appended claims, terms in the singular and the singular forms "a," "an" and "the," for example, optionally include plural referents unless the content clearly dictates otherwise.

The term "subject" as used herein refers to a mammal including human and non-human animals. In one embodiment, the mammal is a human. Terms such as "subject", "patient" or "individual" are terms that can, in context, be used interchangeably in the present disclosure. In certain examples, the subject may be an adult or a child (pediatric) subject.

An "effective amount" refers to at least an amount effective, at dosages and for periods of time necessary, to achieve the desired therapeutic or prophylactic result. An effective amount can be provided in one or more administrations. In some examples of the present disclosure, the term "effective amount" is used to refer to an amount necessary to effect treatment of a disease or condition as hereinbefore described. The effective amount may vary according to the disease or condition to be treated and also according to the weight, age, racial background, sex, health and/or physical condition and other factors relevant to the mammal being treated. Typically, the effective amount will fall within a relatively broad range (e.g. a "dosage" range) that can be determined through routine trial and experimentation by a medical practitioner. The effective amount can be administered in a single dose or in a dose repeated once or several times over a treatment period.

The term "and/or", e.g., "X and/or Y" shall be understood to mean either "X and Y" or "X or Y" and shall be taken to provide explicit support for both meanings or for either meaning.

As used herein, the term about, unless stated to the contrary, refers to +/- 10%, more preferably +/- 5%, of the designated value.

Throughout this specification the word "comprise", or variations such as "comprises" or "comprising", will be understood to imply the inclusion of a stated element, integer or step, or group of elements, integers or steps, but not the exclusion of any other element, integer or step, or group of elements, integers or steps.

### Mesenchymal lineage precursor or stem cells

As used herein, the term "mesenchymal lineage precursor or stem cells" refers to undifferentiated multipotent cells that have the capacity to self-renew while maintaining multipotency and the capacity to differentiate into a number of cell types either of mesenchymal origin, for example, osteoblasts, chondrocytes, adipocytes, stromal cells, fibroblasts and tendons, or non-mesodermal origin, for example, hepatocytes, neural cells and epithelial cells.

The term "mesenchymal lineage precursor or stem cells" includes both parent cells and their undifferentiated progeny. The term also includes mesenchymal precursor cells (MPC), multipotent stromal cells, mesenchymal stem cells, perivascular mesenchymal precursor cells, and their undifferentiated progeny.

Mesenchymal lineage precursor or stem cells can be autologous, allogeneic, xenogeneic, syngeneic or isogeneic. Autologous cells are isolated from the same individual to which they will be reimplanted. Allogeneic cells are isolated from a donor of the same species. Xenogeneic cells are isolated from a donor of another species. Syngeneic or isogeneic cells are isolated from genetically identical organisms, such as twins, clones, or highly inbred research animal models.

Mesenchymal lineage precursor or stem cells reside primarily in the bone marrow, but have also been shown to be present in diverse host tissues including, for example, cord blood and umbilical cord, adult peripheral blood, adipose tissue, trabecular bone and dental pulp.

Mesenchymal lineage precursor or stem cells can be isolated from host tissues and enriched for by immunoselection. For example, a bone marrow aspirate from a subject may be further treated with an antibody to STRO-1 or TNAP to enable selection of mesenchymal lineage precursor or stem cells. In one example, the mesenchymal lineage precursor or stem cells can be enriched for by using the STRO-1 antibody described in Simmons & Torok-Storb, 1991.

STRO-1 + cells are cells found in bone marrow, blood, dental pulp cells, adipose tissue, skin, spleen, pancreas, brain, kidney, liver, heart, retina, brain, hair follicles, intestine, lung, lymph node, thymus, bone, ligament, tendon, skeletal muscle, dermis, and periosteum; and are capable of differentiating into germ lines such as mesoderm and/or endoderm and/or ectoderm. Thus, STRO-1 + cells are capable of differentiating into a large number of cell types including, but not limited to, adipose, osseous, cartilaginous, elastic, muscular, and fibrous connective tissues. The specific lineage-commitment and differentiation pathway which these cells enter depends upon various influences from mechanical influences and/or endogenous bioactive factors, such as growth factors, cytokines, and/or local microenvironmental conditions established by host tissues.

The term "enriched" as used herein describes a population of cells in which the proportion of one particular cell type or the proportion of a number of particular cell types is increased when compared with an untreated population of the cells (e.g., cells in their native environment). In one example, a population enriched for STRO-1+ cells comprises at least about 0.1% or 0.5% or 1% or 2% or 5% or 10% or 15% or 20% or 25% or 30% or 50% or 75% STRO-1 + cells. In this regard, the term "population of cells enriched for STRO-1+ cells" will be taken to provide explicit support for the term "population of cells comprising X% STRO-1+ cells", wherein X% is a percentage as recited herein. The STRO-1+ cells can, in some examples, form clonogenic colonies, for example, CFU-F (fibroblasts) or a subset thereof (e.g., 50% or 60% or 70% or 70% or 90% or 95%) can have this activity. In one example, a population enriched for TNAP+ cells comprises at least about 0.1% or 0.5% or 1% or 2% or 5% or 10% or 15% or 20% or 25% or 30% or 50% or 75% TNAP+ cells. In this regard, the term "population of cells enriched for TNAP+ cells" will be taken to provide explicit support for the term "population of cells comprising X% TNAP+ cells", wherein X% is a percentage as recited herein. In one example, a population enriched for STRO-1+ and TNAP+ cells comprises at least about 0.1% or 0.5% or 1% or 2% or 5% or 10% or 15% or 20% or 25% or 30% or 50% or 75% STRO-1+ and TNAP+ cells. In this regard, the term "population of cells enriched for STRO-1+ and TNAP+ cells" will be taken to provide explicit support for the term "population of cells comprising X% STRO-1+ and TNAP+ cells", wherein X% is a percentage as recited herein.

In one example, the population of cells is enriched from a cell preparation comprising STRO-1+ cells in a selectable form. In this regard, the term "selectable form" will be understood to mean that the cells express a marker (e.g., a cell surface marker) permitting selection of the STRO-1+ cells. The marker can be STRO-1, but need not be. For example, as described and/or exemplified herein, cells (e.g., MPCs) expressing STRO-2 and/or STRO-3 (TNAP) and/or STRO-4 and/or VCAM-1 and/or CD146 and/or 3G5 also express STRO-1 (and can be STRO-1^{bright}). Accordingly, an indication that cells are STRO-1+ does not mean that the cells are selected by STRO-1 expression. In one example, the cells are selected based on at least STRO-3 expression, e.g., they are STRO-3+ (TNAP+).

Reference to selection of a cell or population thereof does not necessarily require selection from a specific tissue source. As described herein, STRO-1+ cells can be selected from or isolated from or enriched from a large variety of sources. That said, in some examples, these terms provide support for selection from any tissue comprising STRO-1+ cells or vascularized tissue or tissue comprising pericytes (e.g., STRO-1 + pericytes) or any one or more of the tissues recited herein.

In one example, the mesenchymal lineage precursor or stem cells of the disclosure express one or more markers individually or collectively selected from the group consisting of TNAP+, VCAM-1+, THY-1+, STRO-2+, STRO-4+ (HSP-90β), CD45+, CD146+, 3G5+.

By "individually" is meant that the disclosure encompasses the recited markers or groups of markers separately, and that, notwithstanding that individual markers or groups of markers may not be separately listed herein, the accompanying claims may define such marker or groups of markers separately and divisibly from each other.

By "collectively" is meant that the disclosure encompasses any number or combination of the recited markers or groups of markers, and that, notwithstanding that such numbers or combinations of markers or groups of markers may not be specifically listed herein, the accompanying claims may define such combinations or sub- combinations separately and divisibly from any other combination of markers or groups of markers.

A cell that is referred to as being "positive" for a given marker may express either a low (lo or dim or dull), intermediate (median) or a high (bright, bri) level of that marker depending on the degree to which the marker is present on the cell surface, where the terms relate to intensity of fluorescence or other marker used in the sorting process of the cells or flow cytometric analysis of the cells. The distinction of low (lo or dim or dull), intermediate (median), or high (bright, bri) will be understood in the context of the marker used on a particular cell population being sorted or analysed. A cell that is referred to as being "negative" for a given marker is not necessarily completely absent from that cell. This term means that the marker is expressed at a relatively very low level by that cell, and that it generates a very low signal when detectably labeled or is undetectable above background levels, for example, levels detected using an isotype control antibody.

The term "bright" or bri as used herein, refers to a marker on a cell surface that generates a relatively high signal when detectably labeled. Whilst not wishing to be limited by theory, it is proposed that "bright" cells express more of the target marker protein (for example, the antigen recognized by a STRO-1 antibody) than other cells in the sample. For instance, STRO-1^{bri} cells produce a greater fluorescent signal, when labeled with a FITC-conjugated STRO-1 antibody as determined by fluorescence activated cell sorting (FACS) analysis, than non-bright cells (STRO-1^{lo/dim/dull/intermediate/median}). In one example, the mesenchymal lineage precursor or stem cells are isolated from bone marrow and enriched for by selection of STRO-1+ cells. In this example, "bright" cells constitute at least about 0.1% of the most brightly labeled bone marrow mononuclear cells contained in the starting sample. In other examples, "bright" cells constitute at least about 0.1%, at least about 0.5%, at least about 1%, at least about 1.5%, or at least about 2%, of the most brightly labeled bone marrow mononuclear cells contained in the starting sample. In an example, STRO-1^{bright} cells have 2 log magnitude higher expression of STRO-1 surface expression relative to "background", namely cells that are STRO-1-. By comparison, STRO-1^{lo/dim/dull} and/or STRO-1^{intermediate/median} cells have less than 2 log magnitude higher expression of STRO-1 surface expression, typically about 1 log or less than "background".

In one example, the STRO-1+ cells are STRO-1^{bright}. In one example, the STRO-1^{bright} cells are preferentially enriched relative to STRO-1^{lo/dim/dull} or STRO-1^{intermediate/median} cells.

In one example, the STRO-1^{bright} cells are additionally one or more of TNAP+, VCAM-1+, THY-1+, STRO-2+, STRO-4+ (HSP-90β) and/or CD146+. For example, the cells are selected for one or more of the foregoing markers and/or shown to express one or more of the foregoing markers. In this regard, a cell shown to express a marker need not be specifically tested, rather previously enriched or isolated cells can be tested and subsequently used, isolated or enriched cells can be reasonably assumed to also express the same marker.

In one example, the STRO-1^{bright} cells are perivascular mesenchymal precursor cells as defined in WO 2004/85630, characterized by the presence of the perivascular marker 3G5.

As used herein the term "TNAP" is intended to encompass all isoforms of tissue non-specific alkaline phosphatase. For example, the term encompasses the liver isoform (LAP), the bone isoform (BAP) and the kidney isoform (KAP). In one example, the TNAP is BAP. In one example, TNAP refers to a molecule which can bind the STRO-3 antibody produced by the hybridoma cell line deposited with ATCC on 19 December 2005 under the provisions of the Budapest Treaty under deposit accession number PTA-7282.

Furthermore, in one example, the STRO-1+ cells are capable of giving rise to clonogenic CFU-F.

In one example, a significant proportion of the STRO-1+ cells are capable of differentiation into at least two different germ lines. Non-limiting examples of the lineages to which the cells may be committed include bone precursor cells; hepatocyte progenitors, which are multipotent for bile duct epithelial cells and hepatocytes; neural restricted cells, which can generate glial cell precursors that progress to oligodendrocytes and astrocytes; neuronal precursors that progress to neurons; precursors for cardiac muscle and cardiomyocytes, glucose-responsive insulin secreting pancreatic beta cell lines. Other lineages include, but are not limited to, odontoblasts, dentin-producing cells and chondrocytes, and precursor cells of the following: retinal pigment epithelial cells, fibroblasts, skin cells such as keratinocytes, dendritic cells, hair follicle cells, renal duct epithelial cells, smooth and skeletal muscle cells, testicular progenitors, vascular endothelial cells, tendon, ligament, cartilage, adipocyte, fibroblast, marrow stroma, cardiac muscle, smooth muscle, skeletal muscle, pericyte, vascular, epithelial, glial, neuronal, astrocyte and oligodendrocyte cells.

In one example, the mesenchymal lineage precursor or stem cells are mesenchymal stem cells (MSCs). The MSCs may be a homogeneous composition or may be a mixed cell population enriched in MSCs. Homogeneous MSC compositions may be obtained by culturing adherent bone marrow or periosteal cells, and the MSCs may be identified by specific cell surface markers which are identified with unique monoclonal antibodies. A method for obtaining a cell population enriched in MSCs using plastic adherence technology is described, for example, in US patent 5486359. MSC prepared by conventional plastic adherence isolation relies on the non-specific plastic adherent properties of CFU-F. Alternative sources for MSCs include, but are not limited to, blood, skin, cord blood, muscle, fat, bone, and perichondrium.

The mesenchymal lineage precursor or stem cells may be cryopreserved prior to administration to a subject.

In a preferred embodiment of the invention, the mesenchymal lineage precursor or stem cells are obtained from a master cell bank derived from mesenchymal lineage precursor or stem cells enriched from the bone marrow of healthy volunteers. The use of mesenchymal lineage precursor or stem cells derived from such a source is particularly advantageous for subjects who do not have an appropriate family member available who can serve as the mesenchymal lineage precursor or stem cell donor, or are in need of immediate treatment and are at high risk of relapse, disease-related decline or death, during the time it takes to generate mesenchymal lineage precursor or stem cells.

The present inventors have shown that mesenchymal precursor cells of the disclosure have unexpectedly high potency in terms of their ability to inhibit T cell proliferation after cryopreservation and thawing. In contrast, prior publications teach that cryopreserved mesenchymal stem cells display impaired immunosuppressive properties following thawing (Francois et al., 2012; Chinnadurai et al., 2016).

The isolated or enriched mesenchymal lineage precursor or stem cells can be expanded ex *vivo* or *in vitro* by culture. As will be appreciated by those skilled in the art, the isolated or enriched mesenchymal lineage precursor or stem cells can be cryopreserved, thawed and subsequently or further expanded *ex vivo* or *in vitro* by culture.

The cultured mesenchymal lineage precursor or stem cells are phenotypically different to cells *in vivo.* For example, in one embodiment they express one or more of the following markers, CD44, NG2, DC146 and CD140b.

The cultured mesenchymal lineage precursor or stem cells are biologically different to cells *in vivo,* having a higher rate of proliferation compared to the largely non-cycling (quiescent) cells *in vivo.*

In one example, a population of cells enriched for mesenchymal lineage precursor or stem cells is seeded at about 6000 to 7000 viable cells/cm² in serum-supplemented culture medium, for example, Dulbecco's Modified Eagle medium (DMEM) supplemented with 10% fetal bovine serum (FBS) and 2mM glutamine, and allowed to adhere to the culture vessel overnight at 37°C, 20% O₂. In an embodiment, the cells are seeded at about 6000, 6100, 6200, 6300, 6400, 6500, 6600, 6700, 6800, 6810, 6820, 6830, 6840, 6850, 6860, 6870, 6880, 6890, 6890, 6900, 6910, 6920, 6930, 6940, 6970, 6980, 6990, or 7000 viable cells/cm², preferably at about 6850 to 6860 viable cells/cm². The culture medium is subsequently replaced and the cells cultured for a total of 68 to 72 hours at 37°C, 5% O₂ prior to co-culturing with T cells and determining the amount of IL-2Rα expressed by the T cells.

### Determining TGFβ1 levels

The present disclosure contemplates any form of assay for determining TGFβ1 levels, including Western blot, enzyme-linked immunosorbent assay (ELISA), fluorescence-linked immunosorbent assay (FLISA), competition assay, radioimmunoassay, lateral flow immunoassay, flow-through immunoassay, electrochemiluminescent assay, nephelometric-based assays, turbidometric-based assay, fluorescence activated cell sorting (FACS)-based assays for detection of TGFβ1 in culture medium used to culture mesenchymal lineage or precursor cells, and surface plasmon resonance (SPR or Biacore).

One form of a suitable assay is, for example, an ELISA or FLISA.

In one form, such an assay involves immobilizing a TGFβ1 binding protein onto a solid matrix, such as, for example a polystyrene or polycarbonate microwell or dipstick, a membrane, or a glass support (e.g., a glass slide). A test sample is then brought into direct contact with the TGFβ1 binding protein and TGFβ1 in the sample is bound or captured. Following washing to remove any unbound protein in the sample, a protein that binds to TGFβ1 at a distinct epitope is brought into direct contact with the captured TGFβ1. This detector protein is generally labelled with a detectable reporter molecule, such as, for example, an enzyme (e.g. horseradish peroxidase (HRP)), alkaline phosphatase (AP) or β-galactosidase) in the case of an ELISA or a fluorophore in the case of a FLISA. Alternatively, a second labeled protein can be used that binds to the detector protein. Following washing to remove any unbound protein the detectable reporter molecule is detected by the addition of a substrate in the case of an ELISA, such as, for example, hydrogen peroxide, TMB, or toluidine, or 5-bromo-4-chloro-3-indol-beta-D-galactopyranoside (x-gal). Of course, the immobilized (capture) protein and the detector protein may be used in the opposite manner.

The level of the antigen in the sample is then determined using a standard curve that has been produced using known quantities of the marker or by comparison to a control sample.

The assays described above are readily modified to use chemiluminescence or electrochemiluminescence as the basis for detection.

As will be apparent to the skilled person, other detection methods based on an immunosorbent assay are useful in the performance of the present disclosure. For example, an immunosorbent method based on the description above using a radiolabel for detection, or a gold label (e.g., colloidal gold) for detection, or a liposome, for example, encapsulating NAD+ for detection or an acridinium linked immunosorbent assay.

In some examples of the disclosure, the level of TGFβ1 is determined using a surface plasmon resonance detector (e.g., BIAcore^{™}, GE Healthcare, Piscataway, N.J.), a flow through device (e.g., as described in US patent 7205159), a micro- or nano-immunoassay device (e.g., as described in US patent 7271007), a lateral flow device (e.g., as described in US publication 20040228761 or US publication 20040265926), a fluorescence polarization immunoassay (FPIA, e.g., as described in US patent 4593089 or US patent 4751190), or an immunoturbidimetric assay (e.g., as described in US patent 5571728 or US patent 6248597).

In one embodiment, the method comprises seeding MLPSCs in a culture vessel at about 50,000 viable cells/cm2.

In one embodiment, the method comprises culturing the MLPSCs in chondrogenic basal medium supplemented with 0.5% bovine serum albumin.

In one embodiment, the method comprises culturing adherent cells for at least 68 to 76 hours. In one embodiment, adherent cells are first obtained by culturing the population of cells overnight in, for example, chondrogenic basal medium supplemented with 0.5% bovine serum albumin, to allow them to adhere to the culture vessel.

In one embodiment, the method comprises collecting a sample of the culture medium in which the MLPSCs were cultured. In one embodiment, the collected sample comprises all of the culture medium in which the cells were cultured.

In one embodiment, the method comprises activating latent TGFβ1 in the culture medium prior to determining the amount of TGFβ1 in the culture medium.

In one embodiment, activating latent TGFβ1 comprises adding an acid, for example, 1 N HCl, to the culture medium to lower the pH of the culture medium. In one embodiment, the method comprises concentrating the culture medium sample prior to lowering the pH. In one embodiment, the method, following addition of the acid, comprises neutralising the pH of the culture medium to 7.2 to 7.6 by adding, for example, 1.2 N NaOH/0.5 M HEPES or 1N NaOH.

In one embodiment, the method comprises determining the amount of TGFβ1 in the culture medium by enzyme-linked immunosorbent assay (ELISA).

In one example, the ELISA comprises:
(i) diluting the culture medium 1:5 in a sample diluent;
(ii) adding the diluted culture medium to a well of a microplate precoated with a monoclonal antibody specific for TGFβ1;
(iii) adding sample diluent to each well of the microplate;
(iv) incubating the microplate for 2 hours at room temperature;
(v) washing the microplate;
(vi) adding TGFβ1 conjugate to the well;
(vii) incubating the microplate for 2 hours at room temperature;
(viii) washing the microplate;
(ix) adding a substrate solution to the well;
(x) incubating the microplate for 30 minutes at room temperature;
(xi) adding a stop solution to the well;
(xii) reading optical density on a microplate reader set to 450 nm with wavelength correction at 570 nm;
(xiii) determining the concentration of TGFβ1 corrected for dilution.

In one embodiment, the sample diluent is chondrogenic basal medium supplemented with 0.5% bovine serum albumin.

In one embodiment, the method further comprising:
preparing serial dilutions of a TGFβ1 standard in a sample diluent with final concentrations ranging from 31.2-2000 pg/ml;
adding the standards to the microplate before step (iii);
constructing a standard curve using a four parameter logistic curve fit; and
determining the concentration of TGFβ1 in the culture medium by reference to the standard curve.

In one embodiment the method for determining the potency of MLPSCs comprises:
(i) obtaining a population of MLPSCs;
(ii) seeding the cells in a culture vessel at 50,000 viable cells/cm2;
(iii) culturing the cells in chondrogenic basal medium supplemented with 0.5% bovine serum albumin;
(iv) collecting the culture medium;
(v) activating latent TGFβ1 released by the cells into the culture medium by adding 1 N HCl to reduce the pH of the culture medium;
(vi) neutralising the pH of the culture medium to 7.2 to 7.6 by adding 1.2 N NaOH/0.5 M HEPES or 1N NaOH;
(vii) diluting the culture medium 1:5 in chondrogenic basal medium supplemented with 0.5% bovine serum albumin;
(viii) adding the diluted culture medium to a well of a microplate precoated with a monoclonal antibody specific for TGFβ1;
(ix) adding sample diluent to each well of the microplate;
(x) incubating the microplate for 2 hours at room temperature;
(xi) washing the microplate;
(xii) adding TGFβ1 conjugate to the well;
(xiii) incubating the microplate for 2 hours at room temperature;
(xiv) washing the microplate;
(xv) adding a substrate solution to the well;
(xvi) incubating the microplate for 30 minutes at room temperature;
(xvii) adding a stop solution to the well;
(xviii) reading optical density on a microplate reader set to 450 nm with wavelength correction at 570 nm;
(xix) determining the concentration of TGFβ1 corrected for dilution.

In one embodiment, the method further comprises:
preparing serial dilutions of a TGFβ1 standard in chondrogenic basal medium supplemented with 0.5% bovine serum albumin with final concentrations ranging from 31.2-2000 pg/ml;
adding the standards to the microplate before step (ix);
constructing a standard curve using a four parameter logistic curve fit; and
determining the concentration of TGFβ1 in the culture medium by reference to the standard curve.

### Compositions and administration

A composition comprising mesenchymal lineage precursor or stem cells may be prepared in a pharmaceutically acceptable carrier. The term "pharmaceutically acceptable carrier" as used herein refers to compositions of matter that facilitate the storage, administration, and/or maintain the biological activity of the mesenchymal lineage precursor or stem cells.

In one example, the carrier does not produce significant local or systemic adverse effect in the recipient. The pharmaceutically acceptable carrier may be solid or liquid. Useful examples of pharmaceutically acceptable carriers include, but are not limited to, diluents, solvents, surfactants, excipients, suspending agents, buffering agents, lubricating agents, adjuvants, vehicles, emulsifiers, absorbants, dispersion media, coatings, stabilizers, protective colloids, adhesives, thickeners, thixotropic agents, penetration agents, sequestering agents, scaffolds, isotonic and absorption delaying agents that do not affect the viability and activity of the mesenchymal lineage precursor or stem cells. The selection of a suitable carrier is within the skill of those skilled in the art.

Suitable pharmaceutical carriers include, but are not limited to, hyaluronan, chemically modified hyaluronan, saline, phosphate buffered saline, chondroitin sulfate, glucosamine, mannosamine, proteoglycan, proteoglycan fragments, chitin, chitosan, or other polysaccharide or polymer material.

Mesenchymal lineage precursor or stem cells can also be incorporated or embedded within scaffolds. Suitable scaffolds include but are not limited to, biological, degradable scaffolds. Natural biodegradable scaffolds include but are not limited to, collagen, fibronectin, and laminin scaffolds. Synthetic biodegradable scaffolds include but are not limited to, polyglycolic acid scaffolds (e.g., as described by (Vacanti, Morse, & Saltzman, 1988) (Cima, Ingber, Vacanti, & Langer, 1991) (Vacanti, Langer , Schloo, & Vacanti, 1991)), synthetic polymers such as, for example, polyanhydrides, polyorthoesters, and polylactic acid; and gelatin resorbable sponges such as, for example, GelformTM (Pfizer).

Compositions of the disclosure may conveniently be presented in unit dosage form and may be prepared by any of the methods well known in the art. The term "dosage unit form" as used herein refers to physically discrete units suited as unitary dosages for subjects to be treated; each unit containing a predetermined quantity of active compound calculated to produce the desired therapeutic or prophylactic effect in association with the pharmaceutical carrier. The dose of mesenchymal lineage precursor or stem cells may vary according to factors such as the disease state, age, sex, and weight of the subject to be treated.

Exemplary doses include at least about 1 x 10⁶ cells. For example, a dose can comprise from about 1.0 x 10⁶ to about 1x10¹⁰ cells, for example, from about 1.1 x 10⁶ to about 1x10⁹ cells, for example, from about 1.2 x 10⁶ to about 1 x 10⁸ cells, for example, from about 1.3 x 10⁶ to about 1 x 10⁷ cells, for example, from about 1.4 x 10⁶ to about 9 x 10⁶ cells, for example, from about 1.5 x 10⁶ to about 8 x 10⁶ cells, for example, from about 1.6 x 10⁶ to about 7 x 10⁶ cells, for example, from about 1.7 x 10⁶ to about 6 x 10⁶ cells, for example, from about 1.8 x 10⁶ to about 5 x 10⁶ cells, for example, from about 1.9 x 10⁶ to about 4 x 10⁶ cells, for example, from about 2 x 10⁶ to about 3 x 10⁶ cells.

In one example, the dose comprises from about 5 x 10⁵ to about 2 x10⁷ cells, for example, from about 6 x 10⁶ cells to about 1.8 x 10⁷ cells. The dose may be, for example, about 6 x 10⁶ cells or about 1.8 x 10⁷ cells.

The mesenchymal lineage precursor or stem cells comprise at least about 5%, at least about 10%, at least about 15%, at least about 20%, at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95% of the cell population of the composition.

Compositions of the disclosure may be cryopreserved. Cryopreservation of mesenchymal lineage precursor or stem cells can be carried out using slow-rate cooling methods or 'fast' freezing protocols known in the art. Preferably, the method of cryopreservation maintains similar phenotypes, cell surface markers and growth rates of cryopreserved cells in comparison with unfrozen cells.

The cryopreserved composition may comprise a cryopreservation solution. The pH of the cryopreservation solution is typically 6.5 to 8, preferably 7.4.

The cryopreservation solution may comprise a sterile, non-pyrogenic isotonic solution such as, for example, PlasmaLyte ATM. 100 mL of PlasmaLyte ATM contains 526 mg of sodium chloride, USP (NaCl); 502 mg of sodium gluconate (C6H11NaO7); 368 mg of sodium acetate trihydrate, USP (C2H3NaO2•3H2O); 37 mg of potassium chloride, USP (KCI); and 30 mg of magnesium chloride, USP (MgCl2•6H2O). It contains no antimicrobial agents. The pH is adjusted with sodium hydroxide. The pH is 7.4 (6.5 to 8.0).

The cryopreservation solution may comprise ProfreezeTM. The cryopreservation solution may additionally or alternatively comprise culture medium, for example, αMEM.

To facilitate freezing, a cryoprotectant such as, for example, dimethylsulfoxide (DMSO), is usually added to the cryopreservation solution. Ideally, the cryoprotectant should be nontoxic for cells and patients, nonantigenic, chemically inert, provide high survival rate after thawing and allow transplantation without washing. However, the most commonly used cryoprotector, DMSO, shows some cytotoxicity. Hydroxylethyl starch (HES) may be used as a substitute or in combination with DMSO to reduce cytotoxicity of the cryopreservation solution.

The cryopreservation solution may comprise one or more of DMSO, hydroxyethyl starch, human serum components and other protein bulking agents. In one example, the cryopreserved solution comprises about 5% human serum albumin (HSA) and about 10% DMSO. The cryopreservation solution may further comprise one or more of methycellulose, polyvinyl pyrrolidone (PVP) and trehalose.

In one embodiment, cells are suspended in 42.5% ProfreezeTM/50% αMEM/7.5% DMSO and cooled in a controlled-rate freezer.

The cryopreserved composition may be thawed and administered directly to the subject or added to another solution, for example, comprising HA. Alternatively, the cryopreserved composition may be thawed and the mesenchymal lineage precursor or stem cells resuspended in an alternate carrier prior to administration.

Compositions of the disclosure can be administered by a route that is suitable for the particular disease state to be treated. For example, compositions of the disclosure can be administered systemically, i.e., parenterally, intravenously or by injection. Compositions of the disclosure can be targeted to a particular tissue or organ.

Dosage regimens may be adjusted to provide the optimum therapeutic response. For example, a single bolus may be administered, several divided doses may be administered over time or the dose may be proportionally reduced or increased as indicated by the exigencies of the therapeutic situation. It may be advantageous to formulate parenteral compositions in dosage unit form for ease of administration and uniformity of dosage.

In some embodiments, it may not be necessary or desirable to immunosuppress a patient prior to initiation of therapy with cellular compositions. However, in other instances it may be desirable or appropriate to pharmacologically immunosuppress a patient prior to initiating cell therapy. This may be accomplished through the use of systemic or local immunosuppressive agents, or it may be accomplished by delivering the cells in an encapsulated device. The cells may be encapsulated in a capsule that is permeable to nutrients and oxygen required by the cell and therapeutic factors the cell is yet impermeable to immune humoral factors and cells. Preferably the encapsulant is hypoallergenic, is easily and stably situated in a target tissue, and provides added protection to the implanted structure. These and other means for reducing or eliminating an immune response to the transplanted cells are known in the art. As an alternative, the cells may be genetically modified to reduce their immunogenicity.

It will be appreciated that the mesenchymal lineage precursor or stem cells may be administered with other beneficial drugs or biological molecules (growth factors, trophic factors). When administered with other agents, they may be administered together in a single pharmaceutical composition, or in separate pharmaceutical compositions, simultaneously or sequentially with the other agents (either before or after administration of the other agents). Bioactive factors which may be co-administered include anti-apoptotic agents (e.g., EPO, EPO mimetibody, TPO, IGF-I and IGF-II, HGF, caspase inhibitors); anti-inflammatory agents (e.g., p38 MAPK inhibitors, TGF-beta inhibitors, statins, IL-6 and IL-1 inhibitors, PEMIROLASTTM, TRANILASTTM, REMICADETM, SIROLIMUSTM, and non-steroidal anti-inflammatory drugs (NSAIDs) such as TEPOXALINTM, TOLMETINTM, SUPROFENTM); immunosupressive/immunomodulatory agents (e.g., calcineurin inhibitors such as cyclosporine, tacrolimus); mTOR inhibitors (e.g., SIROLIMUSTM, EVEROLIMUSTM); anti-proliferatives (e.g., azathioprine, mycophenolate mofetil); corticosteroids (e.g., prednisolone, hydrocortisone); antibodies such as monoclonal anti-IL-2Ralpha receptor antibodies (e.g., basiliximab, daclizumab), polyclonal anti-T-cell antibodies (e.g., anti-thymocyte globulin (ATG); anti-lymphocyte globulin (ALG); monoclonal anti-T cell antibody OKT3)); anti-thrombogenic agents (e.g., heparin, heparin derivatives, urokinase, PPack (dextrophenylalanine proline arginine chloromethylketone), antithrombin compounds, platelet receptor antagonists, anti-thrombin antibodies, anti-platelet receptor antibodies, aspirin, dipyridamole, protamine, hirudin, prostaglandin inhibitors, and platelet inhibitors); and anti-oxidants (e.g., probucol, vitamin A, ascorbic acid, tocopherol, coenzyme Q-10, glutathione, L-cysteine, N-acetylcysteine) as well as local anesthetics.

### Genetically-modified cells

In one embodiment, the mesenchymal lineage precursor or stem cells are genetically unmodified. In one embodiment, the mesenchymal lineage precursor or stem cells are genetically modified, for example, to express and/or secrete a protein of interest, for example, a protein providing a therapeutic and/or prophylactic benefit.

Methods for genetically modifying a cell will be apparent to the skilled person. For example, a nucleic acid that is to be expressed in a cell is operably-linked to a promoter for inducing expression in the cell. For example, the nucleic acid is linked to a promoter operable in a variety of cells of a subject, such as, for example, a viral promoter, for example, a CMV promoter (e.g., a CMV-IE promoter) or a SV-40 promoter. Additional suitable promoters are known in the art.

Preferably, the nucleic acid is provided in the form of an expression construct. The term "expression construct" as used herein refers to a nucleic acid that has the ability to confer expression on a nucleic acid (e.g., a reporter gene and/or a counter-selectable reporter gene) to which it is operably connected, in a cell. Within the context of the present disclosure, it is to be understood that an expression construct may comprise or be a plasmid, bacteriophage, phagemid, cosmid, virus sub-genomic or genomic fragment, or other nucleic acid capable of maintaining and/or replicating heterologous DNA in an expressible format.

Methods for the construction of a suitable expression construct for performance of the invention will be apparent to the skilled person and are described, for example, in Ausubel F. M., 1987 including all updates untill present; or Sambrook & Green, 2012. For example, each of the components of the expression construct is amplified from a suitable template nucleic acid using, for example, PCR and subsequently cloned into a suitable expression construct, such as, for example, a plasmid or a phagemid.

Vectors suitable for such an expression construct are known in the art and/or described herein. For example, an expression vector suitable for the method of the present invention in a mammalian cell is, for example, a vector of the pcDNA vector suite (Invitrogen), a vector of the pCI vector suite (Promega), a vector of the pCMV vector suite (Clontech), a pM vector (Clontech), a pSI vector (Promega), a VP 16 vector (Clontech), or a vector of the pcDNA vector suite (Invitrogen).

The skilled person will be aware of additional vectors and sources of such vectors, such as, for example, Invitrogen Corporation, Clontech or Promega.

Means for introducing the isolated nucleic acid molecule or a gene construct comprising same into a cell for expression are known to those skilled in the art. The technique used for a given organism depends on the known successful techniques. Means for introducing recombinant DNA into cells include microinjection, transfection mediated by DEAE-dextran, transfection mediated by liposomes such as by using lipofectamine (Gibco, MD, USA) and/or cellfectin (Gibco, MD, USA), PEG-mediated DNA uptake, electroporation and microparticle bombardment such as by using DNA-coated tungsten or gold particles (Agracetus Inc., WI, USA) amongst others.

Alternatively, an expression construct of the invention is a viral vector. Suitable viral vectors are known in the art and commercially available. Conventional viral-based systems for the delivery of a nucleic acid and integration of that nucleic acid into a host cell genome include, for example, a retroviral vector, a lentiviral vector or an adeno-associated viral vector. Alternatively, an adenoviral vector is useful for introducing a nucleic acid that remains episomal into a host cell. Viral vectors are an efficient and versatile method of gene transfer in target cells and tissues. Additionally, high transduction efficiencies have been observed in many different cell types and target tissues.

For example, a retroviral vector generally comprises cis-acting long terminal repeats (LTRs) with packaging capacity for up to 6-10 kb of foreign sequence. The minimum cis-acting LTRs are sufficient for replication and packaging of a vector, which is then used to integrate the expression construct into the target cell to provide long term expression. Widely used retroviral vectors include those based upon murine leukemia virus (MuLV), gibbon ape leukemia virus (GaLV), simian immunodeficiency virus (SrV), human immunodeficiency virus (HIV), and combinations thereof (see, e.g., International publication WO1994/026877; Buchschacher & Panganiban, 1992; Johann et al., 1992; Sommerfelt & Weiss, 1990; Wilson et al., 1989; Miller et al., 1991; Lynch, et al., 1991; Miller & Rosman, 1989; Miller, 1990; Scarpa et al., 1991; Burns et al., 1993.

Various adeno-associated virus (AAV) vector systems have also been developed for nucleic acid delivery. AAV vectors can be readily constructed using techniques known in the art. (see, e.g., US patents 5173414 and 5139941; International publications WO 92/01070 and WO 93/03769; Lebkowski et al., 1988; Vincent et al., 1990; Carter, 1992; Muzyczka, 1992; Kotin, 1994; Shelling & Smith, 1994; Zhou et al., 1994.

Additional viral vectors useful for delivering an expression construct of the invention include, for example, those derived from the pox family of viruses, such as vaccinia virus and avian poxvirus or an alphavirus or a conjugate virus vector (e.g., that described in Fisher-Hoch et al., 1989.

### Treatment outcomes

Various method can be used for evaluating the efficacy of treatment of low back pain by administering MLPSCs.

For example, a reduction in lower back pain can be assessed by using the visal analog scale (VAS). The visual analogue scale or visual analog scale (VAS) is a psychometric response scale which can be used in questionnaires. It is a measurement instrument for subjective characteristics or attitudes that cannot be directly measured. When responding to a VAS item, respondents specify their level of agreement to a statement by indicating a position along a continuous line between two end-points. See, for example, Reips, U.-D.; Funke, F (2008). "Interval level measurement with visual analogue scales in Internet-based research: VAS Generator" Behavior Research Methods 40: 699-704.

In another example, a reduction in lower back pain can be assessed by using The Oswestry Disability Index (ODI). This is an index derived from the Oswestry Low Back Pain Questionnaire used by clinicians and researchers to quantify disability for low back pain. This validated questionnaire was first published by Jeremy Fairbank Physiotherapy 1980; 66: 271-273, and subsequently in Fairbank JC, Pynsent PB. The Oswestry Disability Index. Spine 2000 Nov 15;25(22):2940-52.

The self-completed questionnaire contains ten topics concerning intensity of pain, lifting, ability to care for oneself, ability to walk, ability to sit, sexual function, ability to stand, social life, sleep quality, and ability to travel. Each topic category is followed by 6 statements describing different potential scenarios in the patient's life relating to the topic. The patient then checks the statement which most closely resembles their situation. Each question is scored on a scale of 0-5 with the first statement being zero and indicating the least amount of disability and the last statement is scored 5 indicating most severe disability. The scores for all questions answered are summed, then multiplied by two to obtain the index (range 0 to 100). Zero is equated with no disability and 100 is the maximum disability possible.

It will be appreciated by persons skilled in the art that numerous variations and/or modifications may be made to the above-described embodiments, without departing from the broad general scope of the present disclosure. The present embodiments are, therefore, to be considered in all respects as illustrative and not restrictive.

### Examples

### Example 1 Materials and methods

### Mesenchymal Lineage Precursor or Stem Cells (MLPSCs) prepared using plastic adherence techniques

MLPSCs were generated de novo from bone marrow as described in US 5,837,539. Approximately 80-100 ml of bone marrow was aspirated into sterile heparin-containing syringes and taken to the MDACC Cell Therapy Laboratory for MSC generation. The bone marrow mononuclear cells were isolated using ficoll-hypaque and placed into two T175 flasks with 50 ml per flask of MLPSC expansion medium which includes alpha modified MEM (αMEM) containing gentamycin, glutamine (2 mM) and 20% (v/v) fetal bovine serum (FBS) (Hyclone).

The cells were cultured for 2-3 days in 37°C, 5%CO₂ at which time the non-adherent cells were removed; the remaining adherent cells were continually cultured until the cell confluence reached 70% or higher (7-10 days), and then the cells were trypsinized and replaced in six T175 flasks with expansion medium (50 ml of medium per flask).

### Immunoselection of Mesenchymal Lineage Precursor or Stem Cells (MLPSCs)

Bone marrow (BM) was harvested from healthy normal adult volunteers (20-35 years old). Briefly, 40 ml of BM is aspirated from the posterior iliac crest into lithium-heparin anticoagulant-containing tubes.

Bone marrow mononuclear cells (BMMNC) were prepared by density gradient separation using Lymphoprep^{™} (Nycomed Pharma, Oslo, Norway) as previously described by Zannettino et al., 1998. Following centrifugation at 400 x g for 30 minutes at 4°C, the buffy layer is removed with a transfer pipette and washed three times in "HHF", composed of Hank's balanced salt solution (HBSS; Life Technologies, Gaithersburg, MD), containing 5% fetal calf serum (FCS, CSL Limited, Victoria, Australia).

STRO-3+ (or TNAP+) cells were subsequently isolated by magnetic activated cell sorting as previously described by Gronthos & Simmons, 1995; and Gronthos, 2003. Briefly, approximately 1-3 x 10⁸ BMMNC are incubated in blocking buffer, consisting of 10% (v/v) normal rabbit serum in HHF for 20 minutes on ice. The cells are incubated with 200 µl of a 10 µg/ml solution of STRO-3 mAb in blocking buffer for 1 hour on ice. The cells are subsequently washed twice in HHF by centrifugation at 400 x g. A 1/50 dilution of goat anti-mouse γ-biotin (Southern Biotechnology Associates, Birmingham, UK) in HHF buffer is added and the cells incubated for 1 hour on ice. Cells are washed twice in MACS buffer (Ca²⁺ - and Mg²⁺ -free PBS supplemented with 1% BSA, 5 mM EDTA and 0.01% sodium azide) as above and resuspended in a final volume of 0.9 ml MACS buffer.

One hundred µl streptavidin microbeads (Miltenyi Biotec; Bergisch Gladbach, Germany) are added to the cell suspension and incubated on ice for 15 min. The cell suspension is washed twice and resuspended in 0.5 ml of MACS buffer and subsequently loaded onto a mini MACS column (MS Columns, Miltenyi Biotec), and washed three times with 0.5 ml MACS buffer to retrieve the cells which did not bind the STRO-3 mAb (deposited on 19 December 2005 with American Type Culture Collection (ATCC) under accession number PTA-7282 - see International publication WO 2006/108229). After addition of a further 1 ml MACS buffer, the column is removed from the magnet and the TNAP+ cells are isolated by positive pressure. An aliquot of cells from each fraction can be stained with streptavidin-FITC and the purity assessed by flow cytometry.

### Example 2 Effect of TGFβ1 on neuropathic pain.

Immunoselected TNAP+ MPCs are selected for incorporation into a product for the treatment of lower back pain based on a threshold level of secretion of TGFβ1. Previous studies (for example Chen et al., J Clin. Invest 2015; 125(8):3226-3240) have shown:
o neuroinflammation, marked by presence of II-1β, IL-6 and TNF, is implicated in the genesis of neuropathic pain;
o CCI induced upregulation of Iba1, II6 and TNF transcripts in the L4-L5 spinal dorsal horn;
o TGFβ1 and IL-10 secreted from BMSCs may control neuropathic pain through their anti-inflammatory properties.

These studies also showed:
o BMSCs secrete greater amounts of TGFβ1 but not IL-10, in the presence of TNF and LPS.
o TGFβ1 was increased in CSF of CCI mice treated with BMSC, but not controls.
∘ Effects of BMSCs on CCl mice were reversed with neutralization of TGF-β1, but not IL-10.
o Knocked down TGFβ1 expression in BMSCs resulted in a 67% reduction in TGFβ1 release and 55% in TGFβ1 expression in BMSC cultures.
∘ BMSC induced effect was compromised for several days after administration of TGFβ1 knocked down BMSCs.
∘ Exogenous TGFβ1 inhibited acute neuropathic pain.
∘ Exogenous TGFβ1 inhibited chronic neuropathic pain.
∘ TGFβ1 receptor 1 eliminated the effects of TGF-β1.

Previous studies (Tolofari et a; Arthritis Res Ther 12 (2010)) have also shown that Sema3A act as a chemorepellent factor against neurons and blood vessels. Current evidence suggests that sema3A in the AF acts as a repellent to neurons in the healthy IVD and that expression is progressively lost with increasing levels of IVD degeneration.

The results herein show that exposure of Human Annulus Fibrosus Cells to recombinant TGFβ1 enhances Sema3A expression as measured by intracellular flow cytometry (Figure 1). This data indicates that MPCs selected for treatment of lower back pain based on a threshold level of secretion of TGF-β1 can significantly reduce neurite ingrowth through TGF-β1 mediated pathways.

### Example 3 Phase 2 Clinical Trial

### Clinical Study Design

This was a randomized, multicenter study with 4 study arms: 2 receiving different doses of MPCs and 2 receiving different control injections (saline or hyaluronic acid). A saline control was chosen as it was anticipated that the saline would have no pharmacological effect. Hyaluronic acid was used to serve as a vehicle control and to assess any therapeutic effect of hyaluronic acid alone.

Treatment assignment occurred in sequential chronological order according to a master randomization list. Randomization was performed centrally, and the assignments were provided to the 13 participating centers. All subjects and radiographic reviewer[s] at the core laboratory were blinded to the assigned randomized treatment. The study center investigator, associated research staff, and sponsor were not blinded to the randomization assignment.

The study had several exploratory efficacy outcomes based on data collected at 30 days and at 3, 6, 12, 24, and 36 months post-injection. Comparisons of effectiveness outcomes were performed between the 2 MPC dose groups, between each MPC dose group and each control group, and among the 4 study arms.

**Clinical Study Patient Population**

| **Inclusion Criteria** | **Exclusion Criteria** |
|---|---|
| DDD with back pain >6 months | Modified Pfirrmann score of 1 & 2 or 7 & 8 |
| Failed 3 Months Non-Operative Care | Clinically significant nerve or sacroiliac joint pain |
| ODI Score >30 | Clinically significant facet pain as determined by a diagnostic medial branch block or facet joint injection |
| With or without contained disc herniation up to a 3mm protrusion with no radiographic evidence of neurological compression | Symptomatic involvement of more than one lumbar disc level |
| Disc height loss of <30% compared to a normal adjacent disc based upon radiographic evaluation | Discs with full thickness tears with free flowing contrast through the annulus fibrosis |
| VAS Back pain >40 | Lumbar intervertebral foraminal stenosis at the affected level(s) resulting in clinically significant spinal nerve root compression |

### Treatments

Subjects received one of the following four treatments:
- Approximately 6.0 million allogeneic MPCs (1.0 mL of the 30 million/5 mL MPC product) mixed with 1.0 mL of the 1% sodium hyaluronate (Euflexxa^{®});
- Approximately 18.0 million allogeneic MPCs (1.0 mL of the 90 million/5 mL MPC product) mixed with 1.0 mL of the 1% sodium hyaluronate;
- 1% sodium hyaluronate control, 2 mL
- Sterile saline control, 2 mL.

The investigational or control treatments were injected directly into the nucleus pulposus of the target disc with a sterile pressure manometer syringe and a 23-gauge Luer-Lok^{™} needle using a posterior approach under fluoroscopic guidance.

### Investigational Products and administration

The investigational product was STRO-3 selected allogeneic MPCs, which were derived from adult bone marrow mononucleated cells that were culture-expanded and subsequently cryopreserved. The allogeneic MPCs were formulated in concentrations of 30-million and 90-million nucleated cells in a 5mL volume and cryopreserved in 7.5% dimethylsulfoxide/50% alpha modified Eagle's medium and 42.5% ProFreeze^{®}.

The investigational product was stored in the vapor phase of liquid nitrogen at -140°C to - 196°C until ready for use. The investigational product was to be appropriately identified and segregated from other products.

Subjects randomized to receive a total dose of 6-million MPCs received 2.0-mL of a mixture of the 30-million/5 mL cell product mixed 1:1 by volume with hyaluronic acid, which was effectively 1.0-mL of the 30-million/5 mL cell product mixed with 1.0 mL of the hyaluronic acid. Subjects randomized to receive a total dose of 18-million MPCs received 2.0-mL of a mixture of the 90-million/5 mL cell product mixed 1:1 by volume with hyaluronic acid, which was effectively 1.0-mL of the 90-million/5 mL cell product mixed with 1.0 mL of the hyaluronic acid.

The control agents administered were either 2.0 mL of hyaluronic acid alone or 2.0 mL of saline. Centers used sterile saline from their facility's normal supplies. The hyaluronic acid was supplied by Mesoblast.

The volume of injection for each subject randomized to either investigational or control treatment was to be 2.0 mL to avoid potential differences in injection volume affecting the results.

All subjects in this study underwent an injection of investigational product or control into the nucleus pulposus of a single target lumbar disc. Each qualified subject was randomized to 1 of 2 doses of allogeneic MPCs with an equivalent volume of hyaluronic acid, or to 1 of 2 control arms, hyaluronic acid or saline injection.

### Clinical Study Results

### Safety - Serious Adverse Events

Procedure and treatment well tolerated:
▪ No clinical symptoms of allergic or immune reaction to allogeneic MPCs
   - The proportions of subjects with ≥5% and ≥20% class I PRA and class II PRA remained relatively stable from screening to subsequent visits in all 4 study arms
   - The number of subjects with a DSA response was similar across all treatment groups: subjects in the saline, HA, 6M and 18M groups, 3/16 (18.8%); 2/14 (14.3%); 5/23 (21.7%) and 4/21 (19.0%), respectively.
▪ Serious adverse events
   - Saline had 10% of subjects experience an SAE
      - One incidence of fatigue
      - One incidence of back pain
      - One incidence of leg pain
   - HA had 5.0% of subjects experience an SAE
      - One incidence of back pain
   - 6M MPC had 13.3% of subjects experience an SAE
      - One incidence of dermoid cyst
      - One incidence of discitis (procedure related)
      - Two incidences of back pain
   - 18M MPC had 6.7% of subjects experience and SAE
      - Two incidences of back pain

### Interventions Through 24 months

As shown in Table 1 below, MPC treated groups had significantly fewer interventions through 12 months than controls:

**Table 1**

| | **Interventions 0--12 Months** | | | **Interventions 12-24 Months** | | | **Total Through 24 Months** % (n) |
|---|---|---|---|---|---|---|---|
| | **Surgical % (n)** | **Injection % (n)** | **Total % (n)** | **Surgical % (n)** | **Injection % (n)** | **Total % (n)** | |
| **Saline** (n=20) | 5.0% (1) | 20.0% (4) | **25%a,b (5)** | 0.0% (0) | 0.0% (0) | 0.0% (0) | **25%c (5)** |
| **HA** (n=20) | 0.0% (0) | 10.0% (2) | **10.0% (2)** | 0.0% (0) | 10.0% (2) | 10.0% (2) | **20%d (4)** |
| **6M MPCs** (n=30) | 3.3% (1) | 0.0% (0) | **3.3%a (1)** | 3.3% (1) | 6.7% (2) | 10.0% (3) | **13.3% (4)** |
| **18M MPCs** (n=30) | 3.3% (1) | 0.0% (0) | **3.3%b (1)** | 0.0% (0) | 0.0% (0) | 0.0% (0) | **3.3%c,d (1)** |

| Log-Rank Statistics | | Log-Rank Statistics | |
|---|---|---|---|
| a. | p=0.014 6M MPC vs. saline through 12 months | c. | c. p=0.010 18M MPC vs. saline through 24 months |
| b. | p=0.010 18M MPC vs. saline through 12 months | d. | d. p=0.050 18M MPC vs. saline through 24 months |

### LS Mean change in VAS low back pain

MPC treated groups had a statistically superior LS mean low back pain improvement from baseline compared to saline at 12 and 24 months (Figure 2).

### 24 Month VAS Categorical Distribution

Patients treated with 6M MPCs showed a pronounced shift to lower VAS compared to saline control. Median VAS score for 6M MPC is substantially reduced while saline is virtually unchanged at 24 months (Figure 3).

### 12 MonthResults VAS Responder rates by response thresholds

Both MPC groups have higher proportions of patients than either control group who achieved greater the 50% pain reduction at 12 months (Figure 4).

6 million MPC group has a greater proportion of patients through 12 and through 24 months with at least a 50% pain reduction and no post-treatment intervention compared to controls (Figure 5).

### VAS Comparison to Standard of Care

MPC therapy has significant improvement in mean LBP improvement compared to NSAIDs and Opioids as well as exceeds substantial improvement threshold at all times (Figure 6).

### Mean change in ODIfunction

MPCs had a mean ODI improvement of at least the FDA threshold at every timepoint after 1 month and were superior to saline at both 24 and 36 months (Figure 7).

### 24 MonthResults

MPC treated groups had a greater improvement in function than controls at 12 months (Figure 8).

MPC treated groups have a greater proportion of patients through 24 months with at least 15 point ODI reduction compared to controls (Figure 9).

### ODI Comparison to Standard of Care

MPC therapy has significant improvement in mean function improvement compared to NSAIDs and Opioids as well as exceeds FDA improvement threshold at all times (Figure 10).

### Composite Treatment Success by Timepoint

MPC groups show treatment benefit within 3 months and sustained benefit through at least 24 months consistent with a potential regenerative process (Figure 11).

### Composite Treatment Success Through 24 Months

MPC groups show a significant difference in Treatment Success Responders through 12 months compared to saline control and 6M MPCs compared to saline through 24 months (Figure 12).

### References

Ausubel, F. M. (Ed.). (1987 including all updates untill present). Current Protocols in Molecular Biology. New York: John Wiley & Sons.
Brown, T. A. (Ed.). (1991). Essential Molecular Biology: A Practical Approach (Vol. 1 and 2). Oxford: IRL Press at Oxford University Press.
Buchschacher & Panganiban (1992). Journal of Virology, 2731-2739.
Burns et al., (1993). Proceedings of the National Academy of Sciences USA, 8033-8037.
Carter (1992). Current Opinion in Biotechnology, 533-539.
Chinnadurai et al., (2016). Translational and Clinical Research, 34(9), 2429-2442.
Coligan, J. E., Kruisbeek, A. M., Margulies, D. H., Shevach, E. M., & Strober, W. (Eds.). (1991 including all updates until present). Current Protocols in Immunology. New York: John Wiley & Sons.
Fisher-Hoch et al., (1989). Proceedings of the National Academy of Sciences USA, 56, 317-321. Francois et al., (2012). Cytotherapy, 14(2), 147-152.
Glover, M., & Hames, B. D. (Eds.). (1995 and 1996). DNA Cloning: A Practical Approach (Vols. 1-4). Gronthos (2003). Journal of Cell Science, 116(Pt 9), 1827-1835.
Gronthos & Simmons (1995). Blood, 85(4), 929-940.
Harlow, E., & Lane, D. (1988). Antibodies: A Laboratory Manual. New York: Cold Spring Harbor Laboratory Press.
Johann et al., (1992). Journal of Virology, 65, 1635-1640.
Kotin (1994). Human Gene Therapy, 793-801.
Lebkowski et al., (1988). Molecular and Cellular Biology, 3988-3996.
Miller (1990). Human Gene Therapy, 7, 5-14.
Miller & Rosman (1989). Biotechniques, 7, 980-990.
Miller et al., (1991). Journal of Virology, 65, 2220-2224.
Muzyczka (1992). Current Topics in Microbiology and Immunology, 158, 97-129.
Perbal, B. V. (1984). A Practical Guide to Molecular Cloning. New York: Wiley.
Sambrook, J., & Green, M. R. (2012). Molecular Cloning: A Laboratory Manual (Fourth Edition). New York: Cold Spring Harbour Laboratory Press.
Scarpa et al., (1991). Virology, 75, 849-852.
Sommerfelt & Weiss (1990). Virology, 76, 58-59.
Vincent et al., (1990). Vaccine, 353-359.
Wilson et al., (1989). Journal of Virology, 63, 2374-2378.
Zannettino et al., (1998). Blood, 92(8), 2613-2628.

In particular, the present invention pertains to the following:
1. A method of treating lower back pain in a subject in need thereof, the method comprising administering to the subject a composition comprising mesenchymal lineage precursor or stem cells (MLPSCs), wherein the lower back pain is associated with an intervertebral disc that has a disc height that is not substantially reduced compared to that of an adjacent healthy disc in the subject.
2. The method of item 1 wherein the lower back pain is associated with an intervertebral disc that has a disc height loss of <30% compared to that of an adjacent healthy disc in the subject.
3. The method of item 1 or item 2 wherein the lower back pain is non-radicular in origin.
4. The method of any one of items 1 to 3 wherein the lower back pain is associated with an intervertebral disc herniation up to a 3mm protrusion.
5. The method of any one of items 1 to 4 wherein the lower back pain is associated with nerve ingrowth into an intervertebral disc.
6. The method of any one of items 1 to 5 wherein the lower back pain is associated with inflammation in an intervertebral disc.
7. The method of item 5 or item 6 wherein the nerve ingrowth or inflammation is in the intervertebral disc space, or the nucleus pulposus, or the annulus fibrosis of the intervertebral disc.
8. The method of any one of items 1 to 7 wherein the MLPSCs release TGFβ1 when cultured in an amount of at least about 2800 pg/10⁶ cells, or at least about at 2810 pg/10⁶ cells, or at least about 2820 pg/10⁶ cells, or at least about 2830 pg/10⁶ cells, or at least about 2840 pg/10⁶ cells, or at least about 2850 pg/10⁶ cells, or at least about 2860 pg/10⁶ cells, or at least about 2870 pg/10⁶ cells, or at least about 2880 pg/10⁶ cells, or at least about 2890 pg/10⁶ cells, or at least about 2900 pg/10⁶ cells, or at least about 2910 pg/10⁶ cells, or at least about 2920 pg/10⁶ cells, or at least about 2930 pg/10⁶ cell, or at least about 2940 pg/10⁶ cells, or at least about 2950 pg/10⁶ cells, or at least about 2960 pg/10⁶ cells, or at least about 2970 pg/10⁶ cells, or at least about 2980 pg/10⁶ cells, or at least about 2990 pg/10⁶ cells, or at least about 3000 pg/10⁶ cells.
9. The method of any one of items 1 to 7 wherein the MLPSCs release TGFβ1 when cultured in an amount of at least about an amount of at least about 400 pg/ml, or at least about 405 pg/ml, or at least about 410 pg/ml, or at least about 415 pg/ml, or at least about 420 pg/ml, or at least about 425 pg/ml, or at least about 430 pg/ml, or at least about 435 pg/ml, or at least about 440 pg/ml, or at least about 445 pg/ml, or at least about 450 pg/ml, or at least about 455 pg/ml, or at least about 460 pg/ml, or at least about 465 pg/ml, or at least about 470 pg/ml, or at least about 475 pg/, or at least about 480 pg/ml, or at least about 485 pg/ml, or at least about 490 pg/ml, or at least about 495 pg/ml, or at least about 500 pg/ml.
10. The method of item 8 or item 9 wherein MLPSCs release TGFβ1 in an amount sufficient to enhance Sema3A expression in the annulus fibrosis of the intervertebral disc space.
11. The method of any one of items 1 to 10, wherein the mesenchymal lineage precursor or stem cells are isolated by immunoselection.
12. The method of any one of items 1 to 10, wherein the immunoselected cells are culture expanded prior to administration.
13. The method of any one of items 1 to 10, wherein the mesenchymal lineage precursor or stem cells are culture expanded mesenchymal stem cells.
14. The method according to any one of items 1 to 13, wherein the composition is administered to the subject at a dose of between about 1 x 10⁶ cells to about 20 x 10⁶ cells.
15. The method according to any one of items 1 to 14, wherein the composition is administered to the subject at a dose of about 6 x 10⁶ cells.
16. The method according to any one of items 1 to 14, wherein the composition is administered to the subject at a dose of about 18 x 10⁶ cells.
17. The method according to any one of items 1 to 16, wherein the composition is administered as a single dose.
18. The method according to any one of items 1 to 17, wherein the composition is administered into the nucleus pulposus or the annulus fibrosis of an intervertebral disc.
19. The method according to any one of items 1 to 18 wherein administration of the MLSPCs results in at least a 50% reduction in pain as determined by the visual analog scale (VAS) for at least 1 month, or at least 6 months, or at least 12 months, or at least 18 months, or at least 24 months after administration.
20. The method according to any one of items 1 to 19 wherein administration of the MLSPCs results in a reduction of at least 15 points as determined by the Oswestry Disability Index (ODI) for at least 1 month, or at least 6 months, or at least 12 months, or at least 18 months, or at least 24 months after administration.

## Claims

1. A composition comprising allogeneic human STRO-1⁺ TNAP⁺ mesenchymal precursor cells (MPCs) for use in a method of treating lower back pain in a human subject in need thereof, wherein the method comprises administering to the subject the composition comprising the MPCs into the nucleus pulposus or annulus fibrosus of an intervertebral disc, wherein the lower back pain is associated
i) without disc herniation and with no radiographic evidence of neurological compression;
(ii) with a disc height loss of less than 30% compared to a normal adjacent disc based upon radiographic evaluation; and
(iii) with a visual analog scale (VAS) back pain score greater than 40; and wherein administration of the TNAP+ MLSPCs is at a dose sufficient to result in at least a 50% reduction in pain as determined by visual analog scale (VAS) for at least 6 months after administration; and a reduction of at least 15 points as determined by Oswestry Disability Index (ODI) for at least 6 months after administration.

2. The composition for use of claim 1, wherein lower back pain is associated with inflammation in an intervertebral disc.

3. The composition for use of claim 2, wherein the inflammation is in the intervertebral disc space, or the nucleus pulposus, or the annulus fibrosis of the intervertebral disc.

4. The composition for use of any one of claims 1 to 3, wherein the MPCs were selected based on the release of TGFβ1 when cultured in an amount of at least 2800 pg/10⁶ cells, or at least 2810 pg/10⁶ cells, or at least 2820 pg/10⁶ cells, or at least 2830 pg/10⁶ cells, or at least 2840 pg/10⁶ cells, or at least 2850 pg/10⁶ cells, or at least 2860 pg/10⁶ cells, or at least 2870 pg/10⁶ cells, or at least 2880 pg/10⁶ cells, or at least 2890 pg/10⁶ cells, or at least 2900 pg/10⁶ cells, or at least 2910 pg/10⁶ cells, or at least 2920 pg/10⁶ cells, or at least 2930 pg/10⁶ cell, or at least 2940 pg/10⁶ cells, or at least 2950 pg/10⁶ cells, or at least 2960 pg/10⁶ cells, or at least 2970 pg/10⁶ cells, or at least 2980 pg/10⁶ cells, or at least 2990 pg/10⁶ cells, or at least 3000 pg/10⁶ cells.

5. The composition for use of any one of claims 1 to 4, wherein the MPCs are culture expanded MPCs.

6. The composition for use of any one of claims 1 to 5, wherein the composition is administered to the subject at a dose of between 1 x 10⁶ MPCs and 20 x 10⁶ MPCs.

7. The composition for use of to any one of claims 1 to 6, wherein the composition is administered to the subject at a dose of 6 x 10⁶ MPCs.

8. The composition for use of any one of claims 1 to 7, wherein the composition is administered to the subject at a dose of 18 x 10⁶ MPCs.
